# EUROPEAN PATENT APPLICATION

(11) **EP 3 950 055 A1**
(43) Date of publication of application: **09.02.2022**
(21) Application number: 20778596.5
(22) Date of filing: 27.03.2020
(51) Int. Cl.: A61P 1/02, A61P 17/00, A61Q 11/00, A61Q 19/00, A61K 9/08, A61K 8/39, A61K 8/67, A61K 8/86, A61K 47/10, A61K 31/375

(54) **AQUEOUS FORMULATION**

(30) Priority: 27.03.2019 JP 2019060268
(71) Applicant: Senju Pharmaceutical Co., Ltd., Osaka-shi Osaka 541-0048 (JP)
(72) Inventor: SAWA Shirou, Osaka-shi, Osaka 541-0048 (JP)
(74) Representative: Mewburn Ellis LLP
(86) International application number: PCT/JP2020/014128
(87) International publication number: WO 2020/196852

(57) **Abstract**

A stable aqueous preparation containing EPC-K is provided. Furthermore, a method for stabilizing an aqueous preparation containing EPC-K is provided. The aqueous preparation contains (a) L-ascorbic acid dl-α-tocopherol phosphoric acid diester potassium salt and (b) at least one selected from the group consisting of a polyoxyethylene alkylphenyl ether, a polyoxyethylene alkyl ether, and a polyoxyethylene fatty acid ester. The method for stabilizing an aqueous preparation includes a step of preparing an aqueous preparation containing (a) L-ascorbic acid dl-α-tocopherol phosphoric acid diester potassium salt and (b) at least one selected from the group consisting of a polyoxyethylene alkylphenyl ether, a polyoxyethylene alkyl ether, and a polyoxyethylene fatty acid ester.

## Description

### Technical Field

The present invention generally relates to an aqueous preparation, and specifically relates to an aqueous preparation containing L-ascorbic acid dl-α-tocopherol phosphoric acid diester potassium salt (EPC-K).

### Background Art

L-ascorbic acid dl-α-tocopherol phosphoric acid diester potassium salt (hereinafter sometimes referred to as "EPC-K") is a compound in which vitamin C and vitamin E are ester-bonded via phosphoric acid. L-ascorbic acid dl-α-tocopherol phosphoric acid diester potassium salt has an anti-oxidative action, an anti-inflammatory action, and a moisturizing action, and is used in cosmetics such as hair restorers (OHBA Mihoko et al., YAKUGAKU ZASSHI, 114 (7), 514-522 (1994) (Non Patent Literature 1)). Furthermore, L-ascorbic acid dl-α-tocopherol phosphoric acid diester potassium salt is expected to be applied to oral compositions such as mouthwashes, quasidrugs, and medicines such as eye drops and injections.

### Citation List

### Non Patent Literature

Non Patent Literature 1: OHBA Mihoko et al., YAKUGAKU ZASSHI, 114 (7), 514-522 (1994)

### Summary of Invention

### Technical Problem

An object of the present invention is to provide a stable aqueous preparation containing EPC-K. An object of the present invention is also to provide a method for stabilizing an aqueous preparation containing EPC-K.

### Solution to Problem

The present inventors have intensively studied to solve the above-described problem. As a result, it has been found that EPC-K can be stabilized by adding at least one of a polyoxyethylene alkylphenyl ether, a polyoxyethylene alkyl ether, and a polyoxyethylene fatty acid ester to an aqueous preparation containing EPC-K. The present invention is configured as follows based on the above-described finding.

[1] An aqueous preparation containing: (a) L-ascorbic acid dl-α-tocopherol phosphoric acid diester potassium salt; and (b) at least one selected from the group consisting of a polyoxyethylene alkylphenyl ether, a polyoxyethylene alkyl ether, and a polyoxyethylene fatty acid ester.
[2] The aqueous preparation according to the item [1], in which at least one of a polyoxyethylene octylphenyl ether and a polyoxyethylene nonylphenyl ether is contained as the polyoxyethylene alkylphenyl ether.
[3] The aqueous preparation according to the item [1] or [2], in which at least one selected from the group consisting of a polyoxyethylene lauryl ether, a polyoxyethylene cetyl ether, and a polyoxyethylene stearyl ether is contained as the polyoxyethylene alkyl ether.
[4] The aqueous preparation according to any one of the items [1] to [3], in which at least one selected from the group consisting of a polyoxyethylene lauric acid ester, a polyoxyethylene palmitic acid ester, and a polyoxyethylene stearic acid ester is contained as the polyoxyethylene fatty acid ester.
[5] The aqueous preparation according to any one of the items [1] to [4], containing the (b) at least one selected from the group consisting of a polyoxyethylene alkylphenyl ether, a polyoxyethylene alkyl ether, and a polyoxyethylene fatty acid ester at a content of 0.1 w/v% or more.
[6] The aqueous preparation according to any one of the items [1] to [5], containing the L-ascorbic acid dl-α-tocopherol phosphoric acid diester potassium salt at a content of 0.05 w/v% to 0.2 w/v%.
[7] The aqueous preparation according to any one of the items [1] to [6], containing the polyoxyethylene alkylphenyl ether at a content of 0.01 w/v% to 10 w/v%.
[8] The aqueous preparation according to any one of the items [1] to [7], containing the L-ascorbic acid dl-α-tocopherol phosphoric acid diester potassium salt at a content of 0.05 w/v% to 0.1 w/v% and the polyoxyethylene alkylphenyl ether at a content of 0.01 w/v% to 5.0 w/v%.
[9] The aqueous preparation according to any one of the items [1] to [7], containing the L-ascorbic acid dl-α-tocopherol phosphoric acid diester potassium salt at a content of 0.2 w/v% to 1.0 w/v% and the polyoxyethylene alkylphenyl ether at a content of 0.1 w/v% to 10 w/v%.
[10] The aqueous preparation according to any one of the items [1] to [9], containing the polyoxyethylene alkyl ether at a content of 0.1 w/v% to 4.0 w/v%.
[11] The aqueous preparation according to any one of the items [1] to [10], in which the L-ascorbic acid dl-α-tocopherol phosphoric acid diester potassium salt and the polyoxyethylene alkylphenyl ether have a weight ratio of 1 : 1 to 1 : 50.
[12] The aqueous preparation according to any one of the items [1] to [11], in which the L-ascorbic acid dl-α-tocopherol phosphoric acid diester potassium salt and the polyoxyethylene alkyl ether have a weight ratio of 1 : 0.5 to 1 : 20.
[13] The aqueous preparation according to any one of the items [1] to [12], in which the (b) at least one selected from the group consisting of a polyoxyethylene alkylphenyl ether, a polyoxyethylene alkyl ether, and a polyoxyethylene fatty acid ester has a polyoxyethylene chain length of 10 or less.
[14] The aqueous preparation according to any one of the items [1] to [13], in which the (b) at least one selected from the group consisting of a polyoxyethylene alkylphenyl ether, a polyoxyethylene alkyl ether, and a polyoxyethylene fatty acid ester has an HLB of more than 12.
[15] A cosmetic composition containing the aqueous preparation according to any one of the items [1] to [14].
[16] An oral composition containing the aqueous preparation according to any one of the items [1] to [14].
[17] A pharmaceutical composition containing the aqueous preparation according to any one of the items [1] to [14].
[18] A method for stabilizing an aqueous preparation, the method including a step of preparing an aqueous preparation containing: (a) L-ascorbic acid dl-α-tocopherol phosphoric acid diester potassium salt; and (b) at least one selected from the group consisting of a polyoxyethylene alkylphenyl ether, a polyoxyethylene alkyl ether, and a polyoxyethylene fatty acid ester.
[19] The method according to the item [18], in which the polyoxyethylene alkylphenyl ether is at least one of a polyoxyethylene octylphenyl ether and a polyoxyethylene nonylphenyl ether.
[20] The method according to the item [18] or [19], in which the polyoxyethylene alkyl ether is at least one selected from the group consisting of a polyoxyethylene lauryl ether, a polyoxyethylene cetyl ether, and a polyoxyethylene stearyl ether.
[21] The method according to any one of the items [18] to [20], in which the polyoxyethylene fatty acid ester is at least one selected from the group consisting of a polyoxyethylene lauric acid ester, a polyoxyethylene palmitic acid ester, and a polyoxyethylene stearic acid ester.
[22] The method according to any one of the items [18] to [21], in which the aqueous preparation contains the (b) at least one selected from the group consisting of a polyoxyethylene alkylphenyl ether, a polyoxyethylene alkyl ether, and a polyoxyethylene fatty acid ester at a content of 0.1 w/v% or more.
[23] The method according to any one of the items [18] to [22], in which the aqueous preparation contains the L-ascorbic acid dl-α-tocopherol phosphoric acid diester potassium salt at a content of 0.05 w/v% to 0.2 w/v%.
[24] The method according to any one of the items [18] to [23], in which the aqueous preparation contains the polyoxyethylene alkylphenyl ether at a content of 0.01 w/v% to 10 w/v%.
[25] The method according to any one of the items [18] to [24], in which the aqueous preparation contains the L-ascorbic acid dl-α-tocopherol phosphoric acid diester potassium salt at a content of 0.05 w/v% to 0.1 w/v% and the polyoxyethylene alkylphenyl ether at a content of 0.01 w/v% to 5.0 w/v%.
[26] The method according to any one of the items [18] to [24], in which the aqueous preparation contains the L-ascorbic acid dl-α-tocopherol phosphoric acid diester potassium salt at a content of 0.2 w/v% to 1.0 w/v% and the polyoxyethylene alkylphenyl ether at a content of 0.1 w/v% to 10 w/v%.
[27] The method according to any one of the items [18] to [26], in which the aqueous preparation contains the polyoxyethylene alkyl ether at a content of 0.1 w/v% to 4.0 w/v%.
[28] The method according to any one of the items [18] to [27], in which the L-ascorbic acid dl-α-tocopherol phosphoric acid diester potassium salt and the polyoxyethylene alkylphenyl ether have a weight ratio of 1 : 1 to 1 : 50.
[29] The method according to any one of the items [18] to [28], in which the L-ascorbic acid dl-α-tocopherol phosphoric acid diester potassium salt and the polyoxyethylene alkyl ether have a weight ratio of 1 : 0.5 to 1 : 20.
[30] The method according to any one of the items [18] to [29], in which the (b) at least one selected from the group consisting of a polyoxyethylene alkylphenyl ether, a polyoxyethylene alkyl ether, and a polyoxyethylene fatty acid ester has a polyoxyethylene chain length of 10 or less.
[31] The method according to any one of the items [18] to [30], in which the (b) at least one selected from the group consisting of a polyoxyethylene alkylphenyl ether, a polyoxyethylene alkyl ether, and a polyoxyethylene fatty acid ester has an HLB of more than 12.

### Advantageous Effects of Invention

As described above, according to the present invention, it is possible to provide a stable aqueous preparation containing EPC-K. Furthermore, it is possible to provide a method for stabilizing an aqueous preparation containing EPC-K.

### Description of Embodiments

Hereinafter, embodiments of the present invention will be described.

The aqueous preparation according to the present invention contains (a) L-ascorbic acid dl-α-tocopherol phosphoric acid diester potassium salt (EPC-K) and (b) at least one selected from the group consisting of a polyoxyethylene alkylphenyl ether, a polyoxyethylene alkyl ether, and a polyoxyethylene fatty acid ester.

The present invention also relates to a method for stabilizing an aqueous preparation containing EPC-K. The method for stabilizing an aqueous preparation according to the present invention includes a step of preparing an aqueous preparation containing (a) EPC-K and (b) at least one selected from the group consisting of a polyoxyethylene alkylphenyl ether, a polyoxyethylene alkyl ether, and a polyoxyethylene fatty acid ester.

EPC-K easily decomposes in an aqueous solution. For example, Non-Patent Literature 1 describes that when an aqueous solution containing EPC-K had an ethanol concentration in the range of 30 to 70%, the EPC-K was stable. In the case that EPC-K is used as an external preparation for skin such as a hair restorer and that the preparation has a high alcohol concentration, there is a possibility that an allergic symptom, a rash, a rough skin, or a heat sensation will be caused. In the case that EPC-K is used in an eye drop and that the preparation has a high alcohol concentration, there is a possibility that a pain or dysphoria will be caused by an increase in the osmotic pressure or by stimulation of the alcohol. However, in the aqueous preparation according to the present invention, the EPC-K does not necessarily need to be stabilized with an alcohol, so that the aqueous preparation can contain no alcohol or contain an alcohol at a low concentration. The aqueous preparation according to the present invention has reduced irritation to tissues such as skin, and is advantageous.

The aqueous preparation may have any form as long as at least a part of the EPC-K is dissolved in water, and can have a form of, for example, an aqueous solution, an emulsion (milky lotion), or a suspension. Furthermore, the aqueous preparation may be employed for any use, and can be used in a state that the aqueous preparation is contained in, for example, a cosmetic composition, an oral composition, or a pharmaceutical composition. In addition, the aqueous preparation may be used in a state that the aqueous preparation is contained in a cosmetic, a pharmaceutical, or a quasidrug. In the present description, a quasidrug is a product intended for preventing a disease or a disorder, reducing daily discomfort, or managing hygiene. For example, in Japan, the term "quasidrug" refers to a product containing an active ingredient for the effect and efficacy approved by the Ministry of Health, Labor and Welfare. In the present description, examples of a quasidrug may include quasidrugs for all uses, and may include cosmetic compositions, oral compositions, and pharmaceutical compositions. The aqueous preparation can have a form of, for example, an external preparation for skin, a mouthwash, a transdermal preparation, an eye drop, a nasal drop, an oral agent, or an injection. Furthermore, the aqueous preparation can be used in a state that the aqueous preparation is mixed with a paste composition.

The content of the EPC-K in the aqueous preparation depends on the use, and is preferably 0.001 w/v% or more, more preferably 0.01 w/v% or more, and still more preferably 0.05 w/v% or more. The content of the EPC-K in the aqueous preparation is preferably 5.0 w/v% or less, more preferably 2.0 w/v% or less, and still more preferably 0.2 w/v% or less.

The alkyl chain in the polyoxyethylene alkylphenyl ether is not limited, and preferably has 7 or more carbon atoms, and more preferably 8 or more carbon atoms. The alkyl group preferably has 30 or less carbon atoms. The polyoxyethylene chain length in the polyoxyethylene alkylphenyl ether is not limited, and the shorter, the more preferable the polyoxyethylene chain length is. The average addition mole number of the ethylene oxide is preferably 40 or less, more preferably 25 or less, and still more preferably 10 or less. The average addition mole number of the ethylene oxide is 1 or more. As the polyoxyethylene alkylphenyl ether, for example, at least one of a polyoxyethylene octylphenyl ether and a polyoxyethylene nonylphenyl ether is preferably used. As the polyoxyethylene octylphenyl ether, a commercially available product such as Triton X-100 or Triton X-405 can be used.

The alkyl chain in the polyoxyethylene alkyl ether is not limited, and preferably has 18 or less carbon atoms, more preferably 16 or less carbon atoms, and still more preferably 13 or less carbon atoms. The alkyl group has 1 or more carbon atoms. The polyoxyethylene chain length in the polyoxyethylene alkyl ether is not limited, and the shorter, the more preferable the polyoxyethylene chain length is. The average addition mole number of the ethylene oxide is preferably 40 or less, more preferably 25 or less, and still more preferably 10 or less. The average addition mole number of the ethylene oxide is 1 or more. As the polyoxyethylene alkyl ether, for example, at least one selected from the group consisting of a polyoxyethylene lauryl ether, a polyoxyethylene cetyl ether, and a polyoxyethylene stearyl ether can be used, and a polyoxyethylene lauryl ether or a polyoxyethylene cetyl ether is preferably used. As the polyoxyethylene lauryl ether, a commercially available product such as NIKKOL BL-9EX can be used.

The alkyl chain in the polyoxyethylene fatty acid ester is not limited, and preferably has 20 or less carbon atoms, and more preferably 18 or less carbon atoms. The alkyl group has 1 or more carbon atoms, preferably 10 or more carbon atoms, and more preferably 12 or more carbon atoms. The polyoxyethylene chain length in the polyoxyethylene alkyl ether is not limited, and the shorter, the more preferable the polyoxyethylene chain length is. The average addition mole number of the ethylene oxide is preferably 40 or less, and more preferably 25 or less. The average addition mole number of the ethylene oxide is 1 or more, and preferably 10 or more. As the polyoxyethylene fatty acid ester, for example, at least one selected from the group consisting of a polyoxyethylene lauric acid ester, a polyoxyethylene palmitic acid ester, and a polyoxyethylene stearic acid ester can be used, and a polyoxyethylene stearic acid ester or a polyoxyethylene lauric acid ester is preferably used. As the polyoxyethylene stearic acid ester, a commercially available product such as polyethylene glycol monostearate (palmitate and stearate mixture) n = 25: Tokyo Chemical Industry Co., Ltd.) can be used. As the polyoxyethylene lauric acid ester, a commercially available product such as polyethylene glycol monolaurate (n = 10: Tokyo Chemical Industry Co., Ltd.) can be used.

The aqueous preparation preferably contains the at least one selected from the group consisting of a polyoxyethylene alkylphenyl ether, a polyoxyethylene alkyl ether, and a polyoxyethylene fatty acid ester at a content of 0.1 w/v% or more, more preferably more than 0.1 w/v%, and still more preferably 0.5 w/v% or more, and preferably 5 w/v% or less, and more preferably 2 w/v% or less.

The aqueous preparation particularly preferably contains the polyoxyethylene alkylphenyl ether at a content of 0.01 w/v% to 10 w/v%. In the case that the aqueous preparation contains the EPC-K at a content of 0.05 w/v% to 0.1 w/v%, the aqueous preparation preferably contains the polyoxyethylene alkylphenyl ether at a content of 0.01 w/v% to 5 w/v%, and more preferably 0.1 to 2.5 w/v%. In the case that the aqueous preparation contains the EPC-K at a content of 0.2 w/v% to 1.0 w/v%, the aqueous preparation preferably contains the polyoxyethylene alkylphenyl ether at a content of 0.1 w/v% to 10 w/v%, more preferably 0.5 w/v% to 10 w/v%, still more preferably 1.0 w/v% to 10 w/v%, and particularly preferably 2.0 w/v% to 10 w/v%. In the aqueous preparation, the EPC-K and the polyoxyethylene alkylphenyl ether preferably have a weight ratio (the content of the EPC-K : the content of the polyoxyethylene alkylphenyl ether) of 1 : 1 to 1 : 50, and more preferably 1 : 2.5 to 1 : 50.

The aqueous preparation preferably contains the polyoxyethylene alkyl ether at a content of 0.1 w/v% to 4.0 w/v%, and more preferably 0.5 w/v% to 4.0 w/v%. In the aqueous preparation, the EPC-K and the polyoxyethylene alkyl ether preferably have a weight ratio (the content of the EPC-K : the content of the polyoxyethylene alkyl ether) of 1 : 0.5 to 1 : 20, and more preferably 1 : 1 to 1 : 20.

The aqueous preparation preferably contains the polyoxyethylene fatty acid ester at a content of 0.1 w/v% or more.

In the aqueous preparation, the at least one selected from the group consisting of a polyoxyethylene alkylphenyl ether, a polyoxyethylene alkyl ether, and a polyoxyethylene fatty acid ester preferably has an HLB of more than 12.

In such a manner, it is possible to provide a stable aqueous preparation containing EPC-K. In the aqueous preparation according to the present invention, the EPC-K is stabilized without an alcohol, and the aqueous preparation may optionally contain an alcohol. Examples of the alcohol include ethanol and polyols such as butylene glycol, dipropylene glycol, concentrated glycerin, pentylene glycol, propanediol, and isopentyldiol. The content of the alcohol in the aqueous preparation is preferably 40 w/v% or less, more preferably 30 w/v% or less, still more preferably 20 w/v%, and particularly preferably 5 w/v% or less. The lower the content is, the more the irritation is reduced and the more the feeling of use is improved, therefore the more preferable the content is. In the case that the aqueous preparation contains two or more alcohols, the term "content of the alcohol" means the total amount of the two or more alcohols.

In the present description, the term "EPC-K is stabilized" means that after the aqueous preparation containing EPC-K is stored (kept) at a certain temperature for a certain period, the reduced content of the EPC-K is smaller in the aqueous preparation containing the at least one selected from the group consisting of a polyoxyethylene alkylphenyl ether, a polyoxyethylene alkyl ether, and a polyoxyethylene fatty acid ester than in the aqueous preparation containing none of a polyoxyethylene alkylphenyl ether, a polyoxyethylene alkyl ether, and a polyoxyethylene fatty acid ester. In the aqueous preparation according to the present description, the content of the EPC-K is reduced because of the decomposition of the EPC-K. That is, the term "stabilization" of the EPC-K in the aqueous preparation has the same meaning as the term "suppressing the reduction in the content" or "enhancing the residual rate" of the EPC-K in the aqueous preparation. In the present description, the term "residual rate" of the EPC-K in the aqueous preparation means, as described in Examples, the percentage of the content of the EPC-K in the aqueous preparation stored at a certain temperature for a certain period to the content of the EPC-K in the aqueous preparation before the storage.

In the present description, the term "stabilization" of the EPC-K in the aqueous preparation means that the content of the EPC-K in the aqueous preparation is stable before and after the storage at a certain temperature for a certain period (that is, the difference in content is small). More specifically, the residual rate of the EPC-K in the aqueous preparation is preferably maintained at 92% or more, and more preferably at 95% or more after the storage of the aqueous preparation at 60°C for 4 weeks, or the residual rate of the EPC-K in the aqueous preparation is preferably maintained at 92% or more, preferably 94% or more, and more preferably 95% or more after the storage at 40°C for 2 months. Alternatively, in the aqueous preparation according to the present description, the residual rate of the EPC-K in the aqueous preparation is preferably maintained at 85% or more, preferably 90% or more, and more preferably 92% or more after the storage at 40°C for 6 months. The content of the EPC-K in the aqueous preparation can be measured by the method described in Examples shown below.

If necessary, the aqueous preparation can contain an additive that is generally used in an aqueous preparation. Specific examples of the additive include buffers, isotonic agents, solubilizing agents, viscous bases, oily bases, chelating agents, cooling agents, pH adjusting agents, preservatives, antioxidants, and stabilizers.

Examples of the buffer include phosphate buffers, borate buffers, citrate buffers, tartrate buffers, acetate buffers, Tris buffers, and amino acids.

Examples of the isotonic agent include: saccharides such as sorbitol, glucose, and mannitol; polyhydric alcohols such as glycerin and propylene glycol; salts such as sodium chloride; and boric acid.

Examples of the solubilizing agent include polyhydric alcohols such as glycerin and macrogol.

Examples of the viscous base include: water-soluble polymers such as polyvinylpyrrolidone, polyethylene glycol, and a carboxyvinyl polymer; and celluloses such as a hydroxyethyl cellulose, a methyl cellulose, a hydroxypropyl cellulose, a hydroxypropyl methyl cellulose, and a sodium carboxymethyl cellulose.

Examples of the oily base include a castor oil, a soybean oil, a corn oil, and a camellia oil.

Examples of the chelating agent include sodium edetate and citric acid.

Examples of the cooling agent include 1-menthol, borneol, camphor, and a eucalyptus oil.

Examples of the pH adjusting agent include: alkalis such as sodium hydroxide and potassium hydroxide; and acids such as acetic acid, citric acid, hydrochloric acid, phosphoric acid, and tartaric acid.

Examples of the preservative include sorbic acid, potassium sorbate, sodium benzoate, methyl parahydroxybenzoate, ethyl parahydroxybenzoate, propyl parahydroxybenzoate, chlorobutanol, chlorohexidine gluconate, boric acid, dehydroacetic acid, sodium dehydroacetate, benzethonium chloride, benzyl alcohol, zinc chloride, parachlorometaxylenol, chlorocresol, phenethyl alcohol, polidronium chloride, thimerosal, and sodium chlorite.

Examples of the antioxidant include tetrasodium etidronate, disodium edetate, tetrasodium edetate, vitamin C, vitamin E, dibutylhydroxytoluene, butylhydroxyanisole, sodium erythorbate, propyl gallate, sodium sulfite, potassium sulfite, potassium pyrosulfite, sulfur dioxide, chlorogenic acid, catechin, and a rosemary extract.

Examples of the stabilizer include polyvinylpyrrolidone, sodium sulfite, monoethanolamine, glycerin, propylene glycol, cyclodextrins, dextran, ascorbic acid, sodium edetate, taurine, and tocopherol.

The aqueous preparation may contain a surfactant other than a polyoxyethylene alkylphenyl ether, a polyoxyethylene alkyl ether, and a polyoxyethylene fatty acid ester as long as the effect of the present invention and the safety to living bodies are not impaired.

The pH of the aqueous preparation is not particularly limited as long as the pH is within an allowable range depending on the purpose of the aqueous preparation, and the range of 5.5 to 7.0 is preferable. The osmotic pressure of the aqueous preparation is also not particularly limited as long as the osmotic pressure is within an allowable range depending on the purpose. For example, in the case that the aqueous preparation is used as an eye drop, the osmotic pressure ratio to physiological saline of the Japanese Pharmacopoeia is about 0.5 to 3, and preferably about 0.9 to 1.1.

Furthermore, the case will be described in which the aqueous preparation is used as a cosmetic composition or an oral composition.

Examples of the cosmetic composition according to the present invention include compositions for skin, hair, and a scalp. For example, the cosmetic composition can be suitably used in an external preparation for skin or a hair cosmetic such as a hair restorer, a hair growth lotion, a hair growth gel, a hair growth tonic, a hair growth spray, a hair growth tonic spray, a hair tonic, a hair cream, a hair treatment, an antiperspirant, a skin lotion, a shampoo, or a hair dye (hair dyeing agent). In the cosmetic composition, the aqueous preparation can further contain an active ingredient other than EPC-K, or a moisturizer or the like as long as the effect of the present invention and the safety to living bodies are not impaired.

To the cosmetic composition, an active ingredient in a pharmaceutical can be further added. Examples of the active ingredient in a pharmaceutical include minoxidil, finasteride, and carpronium chloride.

To the cosmetic composition, an active ingredient in a quasidrug can be further added. Examples of the active ingredient in a quasidrug include cell activating agents, blood circulation promoters, anti-androgen agents, sebum secretion inhibitors, immunosuppressants, antihistamines, disinfectants, local stimulants, keratin softeners, anti-inflammatory agents, and anti-apoptotic agents, and are not limited thereto. Specifically, one or two or more selected from, for example, carpronium chloride, cantharides tincture, tocopherol (vitamin E) or a derivative thereof, tocopherol acetate, capsicum tincture, a swertia herb extract, a garlic extract, benzyl nicotinate, vitamin A or a derivative thereof, vitamin B 1 or a derivative thereof, vitamin B2 or a derivative thereof, vitamin B6 or a derivative thereof, a dialkyl monoamine derivative, biotin, calcium pantothenate, pantothenyl alcohol, methionine, Calendula officinalis, a ginseng extract, a porphyrin-based compound, a mineral, glyceride of pentadecanoic acid, cystine, serine, leucine, tryptophan, an amino acid extract, a Hypericum perforatum extract, Scutellariae radix, a clove extract, a quachararata extract, a duke extract, eugenyl glucoside, a hop extract, isopropylmethylphenol, biozole, photosensitizer 101, photosensitizer 102, salicylic acid, benzalkonium chloride, benzethonium chloride, chlorhexidine, hinokitiol, phenol, resorcin, sulfur, pyridoxine, lecithin, diethylstilbestrol, thioxolone, diphenhydramine hydrochloride, hydrocortisone acetate, azulene, glycyrrhizin and glycyrrhetinic acid, dipotassium glycyrrhizinate, prednisolone, chondroitin sulphate, hyaluronic acid, pyrrolidonecarboxylic acid, propylene glycol, soluble collagen, glycerin, a mini sasanishiki extract, allantoin, hydrogenated bisabolol, 1-limonene, bisabolol, 1-menthol, delamide, or the like can be used in combination. The cosmetic composition can further contain an additive, and examples of the additive include saw palmetto, zinc, capsaicin, isoflavone, and L-lysine.

The cosmetic composition may also contain a moisturizer. Examples of the moisturizer include pyrrolidone carboxylic acid, hyaluronic acid, a ginseng extract, a tangerine extract, a Citrus junos extract, a marsh mallow extract, a Sanguisorba officinalis extract, a hawthorn extract, a ginkgo biloba extract, a kelp extract, a rice bran extract, rice bran sphingoglycolipid, cacao butter, a common mallow extract, sodium pyrrolidone carboxylate, a pot marigold extract, a royal jelly extract, a lemon extract, a silk extract, an orange extract, an acerola extract, a placenta extract, collagen, a royal jelly extract prune decomposition, a leopard flower extract, a Rehmannia glutinosa extract, a kina extract, a pine extract, a Lamium album flower extract, an Equisetum arvense extract, a Fucus vesiculosus extract, a Chimaphila ymbellata leaf extract, a Zostera marina extract, an aloe extract, a Hypericum perforatum extract, a seaweed extract, chitofilmer, a plant oil (for moisturizing), an Annona cherimola fruit extract, a cucumber fruit extract, a hydrolyzed Hibiscus esculentus extract, Phyllanthus emblica, Acacia concinna, and a Nelumbo nucifera extract (Nelumbo nucifera stamen extract).

The aqueous preparation can be used as an oral composition. The oral composition can be suitably used in a mouthwash, a liquid toothpaste, an oral deodorant, or the like. The aqueous preparation may be mixed in a paste such as a toothpaste. In the oral composition, the aqueous preparation can further contain an active ingredient other than EPC-K, or a sweetener, a colorant, an aroma chemical, a wetting agent, a solvent, a thickener, a pH adjusting agent, a solubilizer, a cleaning agent, a tooth coating agent, a preservative, or the like as long as the effect of the present invention and the safety to living bodies are not impaired.

To the oral composition, an active ingredient in a quasidrug can be further added. Examples of the active ingredient in a quasidrug include bactericides, fluorine-containing compounds (such as sodium fluoride), tranexamic acid, anti-inflammatory agents such as ε-aminocaproic acid, enzymes such as dextranase, vitamins (such as pyridoxine hydrochloride (vitamin B6) and tocopherol (vitamin E)), dipotassium glycyrrhizinate, zeolites, allantoin, and methyl salicylate. Examples of the bactericide include cationic bactericides such as cetylpyridinium chloride, benzethonium chloride, and benzalkonium chloride, nonionic bactericides such as isopropylmethylphenol, and β-glycyrrhetinic acid and sodium lauroylsarcosinate.

Examples of the sweetener include sodium saccharin and stevioside. Examples of the colorant include water-soluble pigments with high safety such as Blue No. 1, Green No. 3, Yellow No. 4, and Red No. 105. Examples of the aroma chemical include natural essential oils such as a peppermint oil, a spearmint oil, a eucalyptus oil, a wintergreen oil, a clove oil, a thyme oil, a sage oil, a cardamom oil, a rosemary oil, a marjoram oil, a lemon oil, a nutmeg oil, a lavender oil, and a paracress oil, aroma components contained in the natural essential oils such as 1-menthol, 1-carvone, cinnamic aldehyde, an orange oil, anethole, 1,8-cineole, methyl salicylate, eugenol, thymol, linalool, limonene, menthone, menthyl acetate, citral, camphor, borneol, pinene, and spilanthol, aroma components such as ethyl acetate, ethyl butyrate, isoamyl acetate, hexanal, hexenal, methyl anthranilate, ethylmethylphenylglycidate, benzaldehyde, vanillin, ethyl vanillin, furaneol, maltol, ethyl maltol, gamma/delta-decalactone, gamma/delta-undecalactone, N-ethyl-p-menthane-3-carboxamide, menthyl lactate, and ethylene glycol-1-menthyl carbonate, and prepared flavors of apple, banana, strawberry, blueberry, melon, peach, pineapple, grape, muscat, wine, cherry, squash, coffee, brandy, yogurt, and the like that include some aroma components and natural essential oils in combination. Examples of the wetting agent include concentrated glycerin, an Equisetum arvense extract, a Crataegus oxyacantha extract, a Hamamelis virginiana extract, a betaine, a Laminaria japonica extract, and sodium hyaluronate, examples of the solvent include ethanol, examples of the thickener include xanthan gum and a hydroxyethyl cellulose, examples of the pH adjusting agent include sodium hydrogen carbonate, sodium hydroxide, sodium citrate, and citric acid, examples of the solubilizer include a POE hydrogenated castor oil, examples of the cleaning agent include alkyl carboxymethyl hydroxyethyl imidazolinium betaine, disodium edetate, and lauryldimethylbetaine, examples of the tooth coating agent include white shellac, and examples of the preservative include phenoxyethanol and paraben.

Next, a method for manufacturing the aqueous preparation according to the present invention will be described. The aqueous preparation can be prepared using a known method. For example, EPC-K, at least one selected from the group consisting of a polyoxyethylene alkylphenyl ether, a polyoxyethylene alkyl ether, and a polyoxyethylene fatty acid ester, and another additive are added to water to prepare an aqueous preparation, and the components of the aqueous preparation can be added in any order.

### [Examples]

Hereinafter, Examples of the present invention will be described. The unit of the content (%) of each component is w/v% unless otherwise specified.

### [Test Example 1]

Samples of the aqueous preparations in Examples and Comparative Examples were prepared in accordance with the prescriptions shown in Table 1. In Example 1, polyoxyethylene (9) lauryl ether having a polyoxyethylene chain length of 9 (NIKKOL BL-9EX, HLB = 14.5) was used as a polyoxyethylene alkyl ether. In Example 2, polyoxyethylene (10) octylphenyl ether having a polyoxyethylene chain length of 10 (Triton X-100, HLB = 13.5) was used as a polyoxyethylene alkylphenyl ether. As EPC-K, EPC (Senju) (Senju Pharmaceutical Co., Ltd.) was used. The surfactants used in Comparative Examples 1 to 6 were polysorbate 80 (HLB = 15), polyoxyethylene hydrogenated castor oil 60 (HLB = 14), polyoxyl monostearate 40 (HLB = 17.5), tyloxapol (HLB = 12.9), and poloxamer 407 (HLB = 18 to 23).

**[Table 1]**

| Prescription | Example 1 | Example 2 | Comparative example 1 | Comparative example 2 | Comparative example 3 | Comparative Example 4 | Comparative Example 5 | Comparative Example 6 |
|---|---|---|---|---|---|---|---|---|
| EPC-K | 0.2 | 0.2 | 0.2 | 0.2 | 0.2 | 0.2 | 0.2 | 0.2 |
| Citric acid | 0.02 | 0.02 | 0.02 | 0.02 | 0.02 | 0.02 | 0.02 | 0.02 |
| Sodium citrate | 0.1 | 0.1 | 0.1 | 0.1 | 0.1 | 0.1 | 0.1 | 0.1 |
| Polyoxyethylene (9) lauryl ether | 1.0 | - | - | - | - | - | - | - |
| Polyoxyethylene (10) octylphenyl ether | - | 1.0 | - | - | - | - | - | - |
| Polysorbate 80 | - | - | - | 1.0 | - | - | - | - |
| Polyoxyethylene hydrogenated castor oil 60 | - | - | - | - | 1.0 | - | - | - |
| Polyoxyl monostearate 40 | - | - | - | - | - | 1.0 | - | - |
| Tyloxapol | - | - | - | - | - | - | 1.0 | - |
| Poloxamer 407 | - | - | - | - | - | - | - | 1.0 |
| Purified water | Appropriate amount | Appropriate amount | Appropriate amount | Appropriate amount | Appropriate amount | Appropriate amount | Appropriate amount | Appropriate amount |
| pH | 5.5 | 5.5 | 5.5 | 5.5 | 5.5 | 5.5 | 5.5 | 5.5 |

Five mL of the obtained sample was put into a 5 mL colorless ampoule and stored at 60°C for 4 weeks, or at 40°C for 2 months or 6 months.

After the storage, the content of the EPC-K was measured. Regarding the content of the EPC-K in the 0.2% EPC-K solution, 2 ml of each sample was precisely measured, and a diluent (50% acetonitrile solution) was added so that the diluted sample had an exact volume of 20 mL. Two mL of this sample was precisely measured, a diluent was added so that the diluted solution had an exact volume of 20 mL to obtain a test solution. Regarding the content of the EPC-K in the 0.1% EPC-K solution, 1 ml of each sample was precisely measured, and a diluent was added so that the diluted sample had an exact volume of 50 mL to obtain a test solution. Regarding the content of the EPC-K in the 0.05% EPC-K solution, 1 ml of each sample was precisely measured, and a diluent was added so that the diluted sample had an exact volume of 25 mL to obtain a test solution. Separately, 10 mg of EPC-K was accurately measured, and a diluent was added so that the diluted solution had an exact volume of 100 mL. Two mL of this solution was precisely measured, a diluent was added so that the diluted solution had an exact volume of 10 mL to obtain a standard solution. A test was performed by liquid chromatography under the conditions described below, the content of the EPC-K was determined at the start of the storage and after the storage, and the percentage of the content of the EPC-K after the storage to the content of the EPC-K at the start of the storage was determined to obtain a residual rate (%).

The test conditions for the liquid chromatography were as follows.
Detector: Ultraviolet absorptiometer (measurement wavelength: 225 nm)
Column: A stainless steel tube having an inner diameter of 4.6 mm and a length of 250 mm is filled with 5 µm of octadecyl silylated silica gel for liquid chromatography. (Capcell pak C18 MG, 5 µm, 4.6 mm × 250 mm, OSAKA SODA CO., LTD.)
Column temperature: A certain temperature around 40°C
Mobile phase: In 150 mL of water, 1.2 g of crystalline sodium dihydrogen phosphate and 0.9 g of sodium chloride were dissolved, and 1,500 mL of methanol and 1,350 mL of acetonitrile were added. To this solution, phosphoric acid was added to adjust the pH to 4.0.
Flow rate: 1.0 mL/min
Inflow: 20 µL

The results are shown in Table 2.

**[Table 2]**

| Prescription | | Example 1 | Example 2 | Comparative example 1 | Comparative example 2 | Comparative example 3 | Comparative Example 4 | Comparative Example 5 | Comparative Example 6 |
|---|---|---|---|---|---|---|---|---|---|
| At the start | Residual rate (%) | 100 | 100 | 100 | 100 | 100 | 100 | 100 | 100 |
| At 60°C for 4 weeks | Residual rate (%) | 94.4 | 95.5 | 85.7 | 67.0 | 89.6 | 91.5 | 90.7 | 91.0 |
| At 40°C for 2 months | Residual rate (%) | 94.1 | 96.0 | 89.5 | - | - | 89.1 | 90.3 | 91.8 |
| At 40°C for 6 months | Residual rate (%) | 88.4 | 92.6 | 77.8 | - | - | 82.0 | 84.9 | - |

As a result of the storage at 60°C, the residual rate of the EPC-K was significantly reduced in Comparative Example 2, compared to Comparative Example 1. In Comparative Examples 3 to 6, a stabilizing effect slightly larger than that in Comparative Example 1 was observed, but the residual rate was about 90% and insufficient. In Examples 1 and 2, the residual rate of the EPC-K was 94% or more, and the stability was improved.

As a result of the storage at 40°C, the residual rates of the EPC-K in Comparative Examples 1, 4, 5 and 6 were almost the same, and no stabilizing effect was observed. In Examples 1 and 2, the reduction in the content of the EPC-K was suppressed, and a significant stabilizing effect was observed.

### [Test Example 2]

Samples of the aqueous preparations in Examples and Comparative Examples were prepared in accordance with the prescriptions shown in Tables 3 to 5. In Examples 3 to 8, polyoxyethylene (9) lauryl ether having a polyoxyethylene chain length of 9 (NIKKOL BL-9EX: FUJIFILM Wako Pure Chemical Corporation, HLB = 14.5) was used as a polyoxyethylene alkyl ether, and the contents varied. Note that the prescriptions in Examples 1 and 5 are the same. In Examples 9 to 22, polyoxyethylene (10) octylphenyl ether having a polyoxyethylene chain length of 10 (Triton X-100: NACALAI TESQUE, INC., HLB = 13.5) was used as a polyoxyethylene alkylphenyl ether, and the contents varied. Note that the prescriptions in Examples 2 and 12 are the same.

**[Table 3]**

| Prescription | Comparative example 1 | Example 3 | Example 4 | Example 5 | Example 6 | Example 7 | Example 8 |
|---|---|---|---|---|---|---|---|
| (a)EPC-K | 0.2 | 0.2 | 0.2 | 0.2 | 0.2 | 0.2 | 0.2 |
| Citric acid | 0.02 | 0.02 | 0.02 | 0.02 | 0.02 | 0.02 | 0.02 |
| Sodium citrate | 0.1 | 0.1 | 0.1 | 0.1 | 0.1 | 0.1 | 0.1 |
| (b) Polyoxyethylene (9) lauryl ether | 0 | 0.1 | 0.5 | 1.0 | 2.0 | 3.0 | 4.0 |
| Purified water | Appropriate amount | Appropriate amount | Appropriate amount | Appropriate amount | Appropriate amount | Appropriate amount | Appropriate amount |
| pH | 5.5 | 5.5 | 5.5 | 5.5 | 5.5 | 5.5 | 5.5 |
| (b)/(a) ratio | 0 | 0.5 | 2.5 | 5 | 10 | 15 | 20 |

**[Table 4]**

| Prescription | Comparative example 1 | Example 9 | Example 10 | Example 11 | Example 12 | Example 13 | Example 14 | Example 15 |
|---|---|---|---|---|---|---|---|---|
| (a)EPC-K | 0.2 | 0.2 | 0.2 | 0.2 | 0.2 | 0.2 | 0.2 | 0.2 |
| Citric acid | 0.02 | 0.02 | 0.02 | 0.02 | 0.02 | 0.02 | 0.02 | 0.02 |
| Sodium citrate | 0.1 | 0.1 | 0.1 | 0.1 | 0.1 | 0.1 | 0.1 | 0.1 |
| (b) Polyoxyethylene (10) octylphenyl ether | 0 | 0.1 | 0.2 | 0.5 | 1.0 | 2.0 | 5.0 | 10.0 |
| Purified water | Appropriate amount | Appropriate amount | Appropriate amount | Appropriate amount | Appropriate amount | Appropriate amount | Appropriate amount | Appropriate amount |
| pH | 5.5 | 5.5 | 5.5 | 5.5 | 5.5 | 5.5 | 5.5 | 5.5 |
| (b)/(a) ratio | 0 | 0.5 | 1 | 2.5 | 5 | 10 | 25 | 50 |

**[Table 5]**

| Prescription | Example 16 | Comparative Example 7 | Example 17 | Example 18 | Example 19 | Example 20 | Example 21 | Example 22 |
|---|---|---|---|---|---|---|---|---|
| (a)EPC-K | 0.1 | 0.05 | 0.05 | 0.05 | 0.05 | 0.05 | 0.05 | 0.05 |
| Citric acid | 0.02 | 0.02 | 0.02 | 0.02 | 0.02 | 0.02 | 0.02 | 0.02 |
| Sodium citrate | 0.1 | 0.1 | 0.1 | 0.1 | 0.1 | 0.1 | 0.1 | 0.1 |
| (b) Polyoxyethylene (10) octylphenyl ether | 1.0 | 0 | 0.025 | 0.05 | 0.125 | 1.25 | 2.5 | 5.0 |
| Purified water | Appropriate amount | Appropriate amount | Appropriate amount | Appropriate amount | Appropriate amount | Appropriate amount | Appropriate amount | Appropriate amount |
| pH | 5.5 | 5.5 | 5.5 | 5.5 | 5.5 | 5.5 | 5.5 | 5.5 |
| (b)/(a) ratio | 10 | 0 | 0.5 | 1 | 2.5 | 25 | 50 | 100 |

The obtained sample was stored in the same manner as in Test Example 1, the content of the EPC-K was measured, and the residual rate of the EPC-K after the storage was determined. The results are shown in Tables 6 to 8.

**[Table 6]**

| Prescription | | Comparative example 1 | Example 3 | Example 4 | Example 5 | Example 6 | Example 7 | Example 8 |
|---|---|---|---|---|---|---|---|---|
| At the start | Residual rate (%) | 100 | 100 | 100 | 100 | 100 | 100 | 100 |
| At 60°C for 4 weeks | Residual rate (%) | 85.7 | 87.3 | 94.5 | 95.1 | 94.8 | 94.7 | 93.5 |
| At 40°C for 2 months | Residual rate (%) | 89.5 | 91.6 | 96.5 | 96.3 | 97.1 | 96.0 | 94.0 |
| At 40°C for 6 months | Residual rate (%) | 77.8 | 80.8 | 89.1 | 90.6 | 91.8 | 92.3 | 89.2 |

**[Table 7]**

| Prescription | | Comparative example 1 | Example 9 | Example 10 | Example 11 | Example 12 | Example 13 | Example 14 | Example 15 |
|---|---|---|---|---|---|---|---|---|---|
| At the start | Residual rate (%) | 100 | 100 | 100 | 100 | 100 | 100 | 100 | 100 |
| At 60°C for 4 weeks | Residual rate (%) | 85.7 | 87.4 | 90.3 | 96.1 | 96.1 | 97.6 | 97.1 | 96.5 |
| At 40°C for 2 months | Residual rate (%) | 89.5 | 92.4 | - | 98.0 | 97.7 | 97.8 | 97.8 | 98.9 |
| At 40°C for 6 months | Residual rate (%) | 77.8 | 82.3 | - | 92.5 | 92.6 | 94.7 | 95.0 | 95.1 |

**[Table 8]**

| | | | | | | | | | |
|---|---|---|---|---|---|---|---|---|---|
| Prescription | Example 16 | Comparative Example 7 | Example 17 | Example 18 | Example 19 | Example 20 | Example 21 | Example 22 | |
| At the start | Residual rate (%) | 100 | 100 | 100 | 100 | 100 | 100 | 100 | 100 |
| At 60°C for 4 weeks | Residual rate (%) | 95.7 | 83.9 | 88.3 | 91.8 | 93.1 | 94.7 | 92.5 | 87.9 |

It was found that in all Examples, the residual rate of the EPC-K in the case of the storage at 60°C was higher than in Comparative Examples 1 and 7, and the stability was improved as shown in Tables 6 to 8. As a result, it was found that the stability of the EPC-K in the aqueous preparation was improved in the case that the content of the polyoxyethylene alkyl ether and the polyoxyethylene alkylphenyl ether was 0.1 w/v% or more.

### [Test Example 3]

Samples of the aqueous preparations in Examples and Comparative Examples were prepared in accordance with the prescriptions shown in Table 9. In Example 23, polyoxyethylene (9) lauryl ether having a polyoxyethylene chain length of 9 (NIKKOL BL-9EX: FUJIFILM Wako Pure Chemical Corporation, HLB = 14.5) was used as a polyoxyethylene alkyl ether. In Example 24, polyoxyethylene (10) octylphenyl ether having a polyoxyethylene chain length of 10 (Triton X-100: NACALAI TESQUE, INC., HLB = 13.5) was used as a polyoxyethylene alkylphenyl ether. In Example 25, polyoxyethylene (23) lauryl ether having a polyoxyethylene chain length of 23 (FUJIFILM Wako Pure Chemical Corporation, HLB = 15.7) was used as a polyoxyethylene alkyl ether. In Example 26, polyoxyethylene (40) octylphenyl ether having a polyoxyethylene chain length of 40 (Triton X-405: NACALAI TESQUE, INC., HLB = 17.9) was used as a polyoxyethylene alkylphenyl ether. Note that the prescriptions in Examples 1 and 23 are the same, and the prescriptions in Examples 2 and 24 are the same.

**[Table 9]**

| Prescription | Comparative example 1 | Example 23 | Example 24 | Example 25 | Example 26 |
|---|---|---|---|---|---|
| EPC-K | 0.2 | 0.2 | 0.2 | 0.2 | 0.2 |
| Citric acid | 0.02 | 0.02 | 0.02 | 0.02 | 0.02 |
| Sodium citrate | 0.1 | 0.1 | 0.1 | 0.1 | 0.1 |
| Polyoxyethylene (9) lauryl ether | - | 1.0 | - | - | - |
| Polyoxyethylene (10) octylphenyl ether | - | - | 1.0 | - | - |
| Polyoxyethylene (23) lauryl ether | - | - | - | 1.0 | - |
| Polyoxyethylene (40) octylphenyl ether | - | - | - | - | 1.0 |
| Purified water | Appropriate amount | Appropriate amount | Appropriate amount | Appropriate amount | Appropriate amount |
| pH | 5.5 | 5.5 | 5.5 | 5.5 | 5.5 |

The obtained sample was stored in the same manner as in Test Example 1, the content of the EPC-K was measured, and the residual rate of the EPC-K after the storage was determined. The results are shown in Table 10.

**[Table 10]**

| Prescription | | Comparative example 1 | Example 23 | Example 24 | Example 25 | Example 26 |
|---|---|---|---|---|---|---|
| At the start | Residual rate (%) | 100 | 100 | 100 | 100 | 100 |
| At 60°C for 4 weeks | Residual rate (%) | 85.7 | 95.1 | 96.1 | 93.7 | 94.4 |
| At 40°C for 2 months | Residual rate (%) | 89.5 | 96.3 | 97.7 | 94.7 | 96.3 |
| At 40°C for 6 months | Residual rate (%) | 77.8 | 90.6 | 92.6 | 90.9 | 92.1 |

As shown in Table 10, the residual rate of the EPC-K after the storage at 60°C for 4 weeks and the residual rate of the EPC-K after the storage at 40°C for 2 months were higher in Examples 23 to 26 than in Comparative Example 1. Furthermore, the residual rate of the EPC-K after the storage was higher in Examples 23 and 24 than in Examples 25 and 26. As a result, it has been found that the stability of the EPC-K can be further improved in the case that the polyoxyethylene alkylphenyl ether or the polyoxyethylene alkyl ether has a polyoxyethylene chain length of 10 or less.

### [Test Example 4]

Samples of the aqueous preparations in Examples and Comparative Examples were prepared in accordance with the prescriptions shown in Table 11. In Example 27, polyoxyethylene (20) cetyl ether having a polyoxyethylene chain length of 20 (FUJIFILM Wako Pure Chemical Corporation, HLB = 17) was used as a polyoxyethylene alkyl ether. In Example 28, polyoxyethylene (20) stearyl ether having a polyoxyethylene chain length of 20 (FUJIFILM Wako Pure Chemical Corporation, HLB = 18) was used as a polyoxyethylene alkyl ether. In Example 29, polyoxyethylene (20) nonylphenyl ether having a polyoxyethylene chain length of 20 (FUJIFILM Wako Pure Chemical Corporation, HLB = 16.2) was used as a polyoxyethylene alkylphenyl ether. In Example 30, polyoxyethylene (25) stearic acid ester having a polyoxyethylene chain length of 25 (Polyethylene Glycol monostearate (palmitate and stearate mixture) n = 25: Tokyo Chemical Industry Co., Ltd., HLB = 15) was used as a polyoxyethylene fatty acid ester. In Example 31, polyoxyethylene (10) lauric acid ester having a polyoxyethylene chain length of 10 (Polyethylene Glycol monolaurate n=10: Tokyo Chemical Industry Co., Ltd., HLB = 12.5) was used as a polyoxyethylene fatty acid ester. The surfactant used in Comparative Example 8 was polyoxyethylene (20) oleyl ether having a polyoxyethylene chain length of 20 (HLB = 17).

**[Table 11]**

| Prescription | Comparative example 1 | Comparative Example 8 | Example 27 | Example 28 | Example 29 | Example 30 | Example 31 |
|---|---|---|---|---|---|---|---|
| EPC-K | 0.2 | 0.2 | 0.2 | 0.2 | 0.2 | 0.2 | 0.2 |
| Citric acid | 0.02 | 0.02 | 0.02 | 0.02 | 0.02 | 0.02 | 0.02 |
| Sodium citrate | 0.1 | 0.1 | 0.1 | 0.1 | 0.1 | 0.1 | 0.1 |
| Polyoxyethylene (20) oleyl ether | - | 1.0 | - | - | - | - | - |
| Polyoxyethylene (20) cetyl ether | - | - | 1.0 | - | - | - | - |
| Polyoxyethylene (20) stearyl ether | - | - | - | 1.0 | - | - | - |
| Polyoxyethylene (20) nonylphenyl ether | - | - | - | - | 1.0 | - | - |
| Polyoxyethylene (25) stearic acid ester | - | - | - | - | - | 1.0 | - |
| Polyoxyethylene (10) lauric acid ester | - | - | - | - | - | - | 1.0 |
| Purified water | Appropriate amount | Appropriate amount | Appropriate amount | Appropriate amount | Appropriate amount | Appropriate amount | Appropriate amount |
| pH | 5.5 | 5.5 | 5.5 | 5.5 | 5.5 | 5.5 | 5.5 |

The obtained sample was stored in the same manner as in Test Example 1, the content of the EPC-K was measured, and the residual rate of the EPC-K after the storage was determined. The results are shown in Table 12.

**[Table 12]**

| Prescription | | Comparative example 1 | Comparative Example 8 | Example 27 | Example 28 | Example 29 | Example 30 | Example 31 |
|---|---|---|---|---|---|---|---|---|
| At the start | Residual rate (%) | 100 | 100 | 100 | 100 | 100 | 100 | 100 |
| At 60°C for 4 weeks | Residual rate (%) | 85.7 | 22.3 | 94.0 | 92.1 | 95.0 | 93.0 | 92.1 |
| At 40°C for 2 months | Residual rate (%) | 89.5 | 35.7 | 95.5 | 91.7 | 98.0 | 96.4 | 95.9 |
| At 40°C for 6 months | Residual rate (%) | 77.8 | 18.3 | 91.5 | 88.1 | 94.8 | 92.8 | 92.8 |

As shown in Table 12, the residual rate of the EPC-K after the storage at 60°C for 4 weeks and the residual rate of the EPC-K after the storage at 40°C for 2 months were higher in Examples 27 to 31 than in Comparative Examples 1 and 8. Furthermore, the residual rate of the EPC-K after the storage was higher in Example 29 than in Examples 27, 28, 30, and 31.

As described above, by adding at least one selected from the group consisting of a polyoxyethylene alkylphenyl ether, a polyoxyethylene alkyl ether, and a polyoxyethylene fatty acid ester to EPC-K, the decomposition of the EPC-K was suppressed at the storage at 60°C and 40°C significantly more than in the case that at least one selected from the group consisting of a polyoxyethylene alkylphenyl ether, a polyoxyethylene alkyl ether, and a polyoxyethylene fatty acid ester was not added. By adding 0.1 w/v% or more of the at least one selected from the group consisting of a polyoxyethylene alkylphenyl ether, a polyoxyethylene alkyl ether, and a polyoxyethylene fatty acid ester, the stabilizing effect was obtained. Furthermore, it has been found that the stabilizing effect is higher in the case that the at least one selected from the group consisting of a polyoxyethylene alkylphenyl ether, a polyoxyethylene alkyl ether, and a polyoxyethylene fatty acid ester has a polyethylene chain length of 10 or less than in the case that the polyethylene chain length is long.

The polyoxyethylene alkylphenyl ether and the polyoxyethylene alkyl ether in Examples were both used at a concentration equal to or higher than the critical micelle concentration (Triton X100: 0.016%, NIKKOL BL-9EX: about 0.26%). Therefore, it is considered that in the aqueous preparations in Examples, the surfactant forms micelles, so that the EPC-K is stabilized. The aqueous preparations prepared in accordance with the prescriptions in Examples had no viscosity visually.

It should be considered that the embodiments and Examples disclosed above are illustrative in all respects and not restrictive. The scope of the present invention is shown not by the above-described embodiments and Examples but by the claims, and includes meanings equivalent to the claims, and includes all modifications and variations within the claims.

## Claims

1. An aqueous preparation comprising:
(a) L-ascorbic acid dl-α-tocopherol phosphoric acid diester potassium salt; and
(b) at least one selected from the group consisting of a polyoxyethylene alkylphenyl ether, a polyoxyethylene alkyl ether, and a polyoxyethylene fatty acid ester.

2. The aqueous preparation according to claim 1, wherein at least one of a polyoxyethylene octylphenyl ether and a polyoxyethylene nonylphenyl ether is contained as the polyoxyethylene alkylphenyl ether.

3. The aqueous preparation according to claim 1 or 2, wherein at least one selected from the group consisting of a polyoxyethylene lauryl ether, a polyoxyethylene cetyl ether, and a polyoxyethylene stearyl ether is contained as the polyoxyethylene alkyl ether.

4. The aqueous preparation according to any one of claims 1 to 3, wherein at least one selected from the group consisting of a polyoxyethylene lauric acid ester, a polyoxyethylene palmitic acid ester, and a polyoxyethylene stearic acid ester is contained as the polyoxyethylene fatty acid ester.

5. The aqueous preparation according to any one of claims 1 to 4, containing the (b) at least one selected from the group consisting of a polyoxyethylene alkylphenyl ether, a polyoxyethylene alkyl ether, and a polyoxyethylene fatty acid ester at a content of 0.1 w/v% or more.

6. The aqueous preparation according to any one of claims 1 to 5, containing the L-ascorbic acid dl-α-tocopherol phosphoric acid diester potassium salt at a content of 0.05 w/v% to 0.2 w/v%.

7. The aqueous preparation according to any one of claims 1 to 6, containing the polyoxyethylene alkylphenyl ether at a content of 0.01 w/v% to 10 w/v%.

8. The aqueous preparation according to any one of claims 1 to 7, containing the L-ascorbic acid dl-α-tocopherol phosphoric acid diester potassium salt at a content of 0.05 w/v% to 0.1 w/v% and the polyoxyethylene alkylphenyl ether at a content of 0.01 w/v% to 5.0 w/v%.

9. The aqueous preparation according to any one of claims 1 to 7, containing the L-ascorbic acid dl-α-tocopherol phosphoric acid diester potassium salt at a content of 0.2 w/v% to 1.0 w/v% and the polyoxyethylene alkylphenyl ether at a content of 0.1 w/v% to 10 w/v%.

10. The aqueous preparation according to any one of claims 1 to 9, containing the polyoxyethylene alkyl ether at a content of 0.1 w/v% to 4.0 w/v%.

11. The aqueous preparation according to any one of claims 1 to 10, wherein the L-ascorbic acid dl-α-tocopherol phosphoric acid diester potassium salt and the polyoxyethylene alkylphenyl ether have a weight ratio of 1 : 1 to 1 : 50.

12. The aqueous preparation according to any one of claims 1 to 11, wherein the L-ascorbic acid dl-α-tocopherol phosphoric acid diester potassium salt and the polyoxyethylene alkyl ether have a weight ratio of 1 : 0.5 to 1 : 20.

13. The aqueous preparation according to any one of claims 1 to 12, wherein the (b) at least one selected from the group consisting of a polyoxyethylene alkylphenyl ether, a polyoxyethylene alkyl ether, and a polyoxyethylene fatty acid ester has a polyoxyethylene chain length of 10 or less.

14. The aqueous preparation according to any one of claims 1 to 13, wherein the (b) at least one selected from the group consisting of a polyoxyethylene alkylphenyl ether, a polyoxyethylene alkyl ether, and a polyoxyethylene fatty acid ester has an HLB of more than 12.

15. A cosmetic composition comprising the aqueous preparation according to any one of claims 1 to 14.

16. An oral composition comprising the aqueous preparation according to any one of claims 1 to 14.

17. A pharmaceutical composition comprising the aqueous preparation according to any one of claims 1 to 14.

18. A method for stabilizing an aqueous preparation, the method comprising a step of preparing an aqueous preparation containing:
(a) L-ascorbic acid dl-α-tocopherol phosphoric acid diester potassium salt; and
(b) at least one selected from the group consisting of a polyoxyethylene alkylphenyl ether, a polyoxyethylene alkyl ether, and a polyoxyethylene fatty acid ester.

19. The method according to claim 18, wherein the polyoxyethylene alkylphenyl ether is at least one of a polyoxyethylene octylphenyl ether and a polyoxyethylene nonylphenyl ether.

20. The method according to claim 18 or 19, wherein the polyoxyethylene alkyl ether is at least one selected from the group consisting of a polyoxyethylene lauryl ether, a polyoxyethylene cetyl ether, and a polyoxyethylene stearyl ether.

21. The method according to any one of claims 18 to 20, wherein the polyoxyethylene fatty acid ester is at least one selected from the group consisting of a polyoxyethylene lauric acid ester, a polyoxyethylene palmitic acid ester, and a polyoxyethylene stearic acid ester.

22. The method according to any one of claims 18 to 21, wherein the aqueous preparation contains the (b) at least one selected from the group consisting of a polyoxyethylene alkylphenyl ether, a polyoxyethylene alkyl ether, and a polyoxyethylene fatty acid ester at a content of 0.1 w/v% or more.

23. The method according to any one of claims 18 to 22, wherein the aqueous preparation contains the L-ascorbic acid dl-α-tocopherol phosphoric acid diester potassium salt at a content of 0.05 w/v% to 0.2 w/v%.

24. The method according to any one of claims 18 to 23, wherein the aqueous preparation contains the polyoxyethylene alkylphenyl ether at a content of 0.01 w/v% to 10 w/v%.

25. The method according to any one of claims 18 to 24, wherein the aqueous preparation contains the L-ascorbic acid dl-α-tocopherol phosphoric acid diester potassium salt at a content of 0.05 w/v% to 0.1 w/v% and the polyoxyethylene alkylphenyl ether at a content of 0.01 w/v% to 5.0 w/v%.

26. The method according to any one of claims 18 to 24, wherein the aqueous preparation contains the L-ascorbic acid dl-α-tocopherol phosphoric acid diester potassium salt at a content of 0.2 w/v% to 1.0 w/v% and the polyoxyethylene alkylphenyl ether at a content of 0.1 w/v% to 10 w/v%.

27. The method according to any one of claims 18 to 26, wherein the aqueous preparation contains the polyoxyethylene alkyl ether at a content of 0.1 w/v% to 4.0 w/v%.

28. The method according to any one of claims 18 to 27, wherein the L-ascorbic acid dl-α-tocopherol phosphoric acid diester potassium salt and the polyoxyethylene alkylphenyl ether have a weight ratio of 1 : 1 to 1 : 50.

29. The method according to any one of claims 18 to 28, wherein the L-ascorbic acid dl-α-tocopherol phosphoric acid diester potassium salt and the polyoxyethylene alkyl ether have a weight ratio of 1 : 0.5 to 1 : 20.

30. The method according to any one of claims 18 to 29, wherein the (b) at least one selected from the group consisting of a polyoxyethylene alkylphenyl ether, a polyoxyethylene alkyl ether, and a polyoxyethylene fatty acid ester has a polyoxyethylene chain length of 10 or less.

31. The method according to any one of claims 18 to 30, wherein the (b) at least one selected from the group consisting of a polyoxyethylene alkylphenyl ether, a polyoxyethylene alkyl ether, and a polyoxyethylene fatty acid ester has an HLB of more than 12.
